(19) Europäisches Patentamt
European Patent Office
Office européen des brevets

(11) EP 0 659 184 B1

(12) **FASCICULE DE BREVET EUROPEEN**

(45) Date de publication et mention de la délivrance du brevet:
**28.02.1996 Bulletin 1996/09**

(21) Numéro de dépôt: **93919419.7**

(22) Date de dépôt: **06.09.1993**

(51) Int Cl.[6]: **C07D 453/04**, C07C 309/24

(86) Numéro de dépôt international:
**PCT/FR93/00848**

(87) Numéro de publication internationale:
**WO 94/06792 (31.03.1994 Gazette 1994/08)**

# (54) (AMINO-3 PHENYL)-1 ETHANESULFONATE D'HYDROQUINININE OPTIQUEMENT ACTIF, SA PREPARATION ET SON UTILISATION

OPTISCH AKTIVES HYDROCHININ (AMINO-3-PHENYL)-1-ETHANSULFONAT, DEREN HERSTELLUNG UND VERWENDUNG

OPTICALLY ACTIVE HYDROQUININE (AMINO-3 PHENYL)-1 ETHANESULFONATE, PREPARATION AND USE THEREOF

(84) Etats contractants désignés:
**AT BE CH DE DK ES FR GB GR IE IT LI LU NL PT SE**

(30) Priorité: **11.09.1992 FR 9210839**

(43) Date de publication de la demande:
**28.06.1995 Bulletin 1995/26**

(73) Titulaire: **RHONE-POULENC RORER S.A.**
**F-92160 Antony (FR)**

(72) Inventeur: **GUYON, Claude**
**F-94100 Saint-Maur-des-Fossés (FR)**

(74) Mandataire: **Savina, Jacques et al**
**F-92165 Antony Cédex (FR)**

(56) Documents cités:
**WO-A-91/12264**
**WO-A-91/13874**
**WO-A-91/13907**
**FR-A- 2 678 938**
**FR-A- 2 682 381**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

EP 0 659 184 B1

Printed by Jouve, 75001 PARIS (FR)

## Description

La présente invention concerne l'(amino-3 phényl)-1 éthanesulfonate d'hydroquinine optiquement actif (Forme A), sa préparation et son utilisation comme intermédiaire dans la préparation d'énantiomères utiles comme antagonistes de la cholécystokinine et de la gastrine.

L'(amino-3 phényl)-1 éthanesulfonate d'hydroquinine (Forme A) peut être préparé selon le procédé suivant:

a) action d'un sulfite de métal alcalin sur le (bromo-1 éthyl)-1 nitro-3 benzène-(RS) et, si nécessaire, passage au sel de potassium pour obtenir le (nitro-3 phényl)-1 éthanesulfonate de potassium-(RS),

b) conversion du (nitro-3 phényl)-1 éthanesulfonate de potassium-(RS) en (nitro-3 phényl)-1 éthanesulfonate de benzylquininium et séparation de la Forme A,

c) réduction du (nitro-3 phényl)-1 éthanesulfonate de benzylquininium forme A.

Il est particulièrement avantageux d'effectuer l'étape a) en milieu aqueux, à une température comprise entre 50°C et 100°C et de préférence à 80°C.

Le sulfite de métal alcalin est de préférence le sulfite de sodium ou de potassium.

Il est préférable pour récupérer le produit de le tranformer en sel de tétra-alkyl ammonium ou de trialkylphénylalkylammonium, l'isoler puis de repasser sous forme de sel de potassium.

L'étape b) s'effectue au moyen d'un halogénure de N-benzyl quininium et notamment le chlorure, en présence de dihydrogénophosphate de potassium, en milieu aqueux, à une température comprise entre 10 et 30°C et de préférence de 20°C.

La réduction de l'étape c) est généralement effectuée au moyen d'hydrogène sous pression, en présence d'un catalyseur tel que du palladium, à une température comprise entre 10 °C et 30°C. Généralement on utilise l'hydrogène sous une pression de 100 kPa.

Le (bromo-1 éthyl)-1 nitro-3 benzène-(RS) peut être préparé selon la méthode décrite par E. FELDER et Coll., J. Med. Chem., 13, 559 (1970).

L'(amino-3 phényl)-1 éthanesulfonate d'hydroquinine (Forme A) est particulièrement intéressant comme intermédiaire pour la préparation des énantiomères des composés de formule :

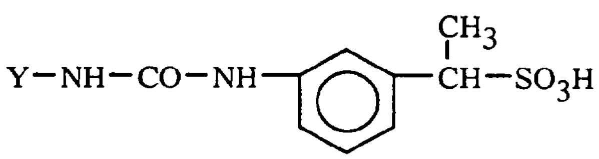

(I)

dans laquelle Y représente :

A) un reste $-CH_2-CO-NR_1R_2$ dans lequel $R_1$ représente un radical (a) phényle éventuellement substitué par un ou plusieurs substituants choisis parmi les atomes d'halogène et les radicaux alkyle, alcoxy, hydroxy polyfluoroalkyle, nitro, alkylthio, alcoxycarbonyle, carboxy, acylamino, méthylènedioxy, polyfluoroalcoxy, trifluorométhylthio, phénoxy, phényl, benzyle, phénylamino et $CONR_3R_4$ dans lequel $R_3$ et $R_4$, identiques ou différents, représentent un atome d'hydrogène, un radical alkyle, phényle (éventuellement substitué par un ou plusieurs substituants choisis parmi les atomes d'halogène et les radicaux alkyle, alcoxy et alkylthio), indanyle, cycloalkylalkyle, cycloalkyle, phénylalkyle ou bien $R_3$ et $R_4$ forment ensemble avec l'atome d'azote auquel ils sont rattachés un hétérocycle mono ou polycyclique saturé ou insaturé contenant 4 à 9 atomes de carbone et un ou plusieurs hétéroatomes (O,N,S) et éventuellement substitué par un plusieurs radicaux alkyle, alcoxy, alcoxycarbonyle, dialkylcarbamoyle, phényle ou en combinaison avec un atome de carbone de l'hétérocycle par un cycle spiromonocyclique à 4 ou 5 chaînons et contenant éventuellement un ou plusieurs hétéroatomes (O,S,N) ; (b) pyridyle, (c) isoquinolyle, (d) quinolyle, (e) quinoxalinyle (ces hétérocycles étant éventuellement substitués par un ou plusieurs substituants choisis parmi les radicaux alkyle, phényle et les atomes d'halogène), (f) alkyle, (g) phénylalkyle, (h) naphtyle, (i) tétrahydro-5,6,7,8 naphtyle, (j) tétrahydro-1,2,3,4 naphtyle, (k) alcoxycarbonylalkyle ou (1) cycloalkyle,
$R_2$ représente une chaîne $-CH(R_5)-CO-R_6$ dans laquelle $R_5$ représente un atome d'hydrogène ou un radical alkyle, alcoxycarbonyle ou phényle (éventuellement substitué par un ou plusieurs substituants choisis parmi les atomes d'halogène et les radicaux alkyle, alcoxy, alkylthio, nitro et amino) et $R_6$ représente un radical alcoxy, cycloalkyloxy (éventuellement substitué par au moins un radical alkyle), cycloalkylalkyloxy, phénylalkyloxy, polyfluoroalkyloxy, cinnamyloxy et $-NR_3R_4$,

B) un reste de formule :

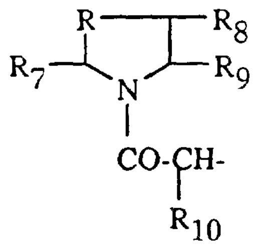

dans laquelle :

- soit R représente un radical méthylène, éthylène, SO, $SO_2$, CHOH ou un atome de soufre, $R_7$ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, $-CO-NR_{11}R_{12}$, $-NH-CO-CH_3$, trifluorométhyle ou trifluorométhoxy et $R_8$ représente un atome d'hydrogène,
- soit R représente un radical méthylène, $R_7$ représente un atome d'hydrogène et $R_8$ représente un radical phényle,
- soit R représente un radical $CHR_{13}$, $R_7$ et $R_8$ représentent chacun un atome d'hydrogène,
- $R_9$ représente un radical alcoxycarbonyle, cycloalkyloxycarbonyle, cycloalkylalkyloxycarbonyle, $-CONR_{14}R_{15}$ ou phényle éventuellement substitué par un ou plusieurs substituants choisis parmi les radicaux alkyle, alcoxy ou hydroxy,
- $R_{10}$ représente un atome d'hydrogène ou un radical alkyle,
- $R_{13}$ représente un radical phényle,
- $R_{11}$ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- $R_{12}$ représente un radical alkyle, phénylalkyle ou phényle éventuellement substitué par un ou plusieurs substituants choisis parmi les atomes d'halogène et les radicaux alkyle, alcoxy et alkylthio,

ou bien $R_{11}$ et $R_{12}$ forment avec l'atome d'azote auquel ils sont rattachés un hétérocycle mono ou polycyclique saturé ou insaturé contenant 4 à 9 atomes de carbone et un ou plusieurs hétéroatomes (O,N) et éventuellement substitué par un ou plusieurs radicaux alkyle,

- $R_{14}$ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- $R_{15}$ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,

ou bien $R_{14}$ et $R_{15}$ forment ensemble avec l'atome d'azote auquel ils sont rattachés un hétérocycle mono ou polycyclique saturé ou insaturé contenant 4 à 9 atomes de carbone et un ou plusieurs hétéroatomes (O,N,S) et éventuellement substitué par un ou plusieurs radicaux alkyle,

C) un reste de formule :

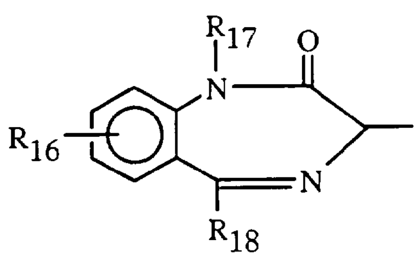

dans laquelle :

- $R_{16}$ représente un atome d'hydrogène ou d'halogène ou un radical alkyle, alkylthio, nitro, hydroxy ou cyano,
- $R_{17}$ représente un radical alkyle ou un reste -CH($R_5$)-CO-$R_6$,
- $R_{18}$ représente un radical pyridyle ou phényle éventuellement substitué par un ou plusieurs substituants choisis parmi les atomes d'halogène et les radicaux alkyle, alcoxy, hydroxy, carboxy et nitro.

Dans ces définitions, les radicaux alkyle et alcoxy et les portions alkyle et alcoxy contiennent de préférence 1 à 4 atomes de carbone en chaîne droite ou ramifiée, les radicaux et portions cycloalkyle 3 à 6 atomes de carbone et les radicaux acyle 2 à 4 atomes de carbone.

Ces composés sont décrits dans les demandes de brevets WO 91/12264, WO 91/13907, WO 91/13874, FR 9108675 et FR 9112481 comme antagonistes de la cholécystokinine et de la gastrine.

Les composés de formule (I) peuvent être préparés à partir de l'(amine-3 phényl)-1 éthanesulfonate d'hydroquinine forme A en opérant de la manière suivante:

on fait agir l'(amino-3 phényl)-1 éthanesulfonate d'hydroquinine forme A sur un dérivé de formule :

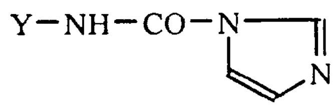

(II)

dans laquelle Y a les mêmes significations que dans la formule (I).

Cette réaction s'effectue généralement au sein d'un solvant inerte tel que le tétrahydrofuranne, le diméthylformamide, un solvant chloré, un solvant aromatique (benzène, toluène par exemple), à une température comprise entre 20°C et la température d'ébullition du solvant.

Les dérivés de formule (II) peuvent être préparés selon les procédés décrits dans les demandes de brevets WO 91/12264, W091/13907, WO 91/13874, FR 9108675 et FR 9112481.

**EXEMPLE 1**

a) (nitro-3 phényl)-1 éthanesulfonate de potassium-(RS):

A une solution de 20,8 g de sulfite de sodium dans 260 $cm^3$ d'eau on ajoute 25,3 g de (bromo-1 éthyl)-1 nitro-3 benzène-(RS). Le mélange réactionnel est agité à 80°C pendant 5 heures, refroidi à environ 25°C et coulé dans 2,5 litres d'une solution aqueuse de dihydrogénophosphate de potassium 0,5 M. On ajoute 40 g d'hydrogénosulfate de tétra-n-butyl ammonium. Le mélange est extrait par 3 fois 500 $cm^3$de chlorure de méthylène. Les phases organiques réunies sont lavées par 2 fois 500 $cm^3$ d'eau, séchées sur sulfate de magnésium et concentrées à sec sous pression réduite (2,7 kPa) à 40°C. L'huile obtenue est dissoute dans 65 $cm^3$ d'acétone et on ajoute 34 g de nonafluorobutanesulfonate de potassium en solution dans 75 $cm^3$ d'acétone. Le produit insoluble est séparé par filtration, lavé par 3 fois 50 $cm^3$ d'oxyde de diisopropyle et séché à l'air. On obtient ainsi 22,4 g de (nitro-3 phényl)-1 éthanesulfonate de potassium-(RS), fondant à une température supérieure à 260°C et utilisé tel quel dans les synthèses ultérieures.

Spectre de R.M.N. : (200 MHz; DMSO d)
δ(ppm) :

1,50 [d, J=7 Hz, 3H: -CH (C$\underline{H}_3$)-]
3,93 [q, J=7 Hz, 1H: -C$\underline{H}$($CH_3$)-]
7,59 [t, J=8 Hz, 1H: -$C_6H_4$(-$\underline{H}$5)]
7,83 [d, J=8 Hz, 1H: -$C_6H_4$(-$\underline{H}$6)]
8,10 [d large,J=8 Hz, 1H: -$C_6H_4$(-$\underline{H}$4)]
8,26 [s large, 1H: -$C_6H_4$(-$\underline{H}$2)].

Le (bromo-1 éthyl)-1 nitro-3 benzène-(RS) peut être préparé selon la méthode décrite par E. FELDER et coll., J. Med. Chem., <u>13</u>, 559 (1970).

b) (nitro-3 phényl)-1 éthanesulfonate de N-benzylquininium, forme A :

A une solution de 17,2 g de (nitro-3 phényl)-1 éthanesulfonate de potassium-(RS) dans 400 $cm^3$ d'eau, on ajoute 87 g de dihydrogénophosphate de potassium et 32,4 g de chlorure de N-benzyl quininium. Le mélange est extrait par 2 fois 300 $cm^3$ de chlorure de méthylène. Les phases organiques réunies sont lavées par 2 fois 200 $cm^3$ d'eau, séchées sur sulfate de magnésium et concentrées à sec sous pression réduite (2,7 kPa)) à 40°C. La meringue

obtenue est dissoute dans 120 $cm^3$ de propanol-2 à reflux. Après refroidissement, les cristaux sont séparés par filtration, lavés par 2 fois 15 $cm^3$ de propanol-2. On obtient, après 2 recristallisations dans 350 puis 500 $cm^3$ de propanol-2, 15,6 g de (nitro-3 phényl)-1 éthanesulfonate de N-benzylquininium, forme A, fondant à environ 110°C, $[\alpha]_D^{20}$ = -151,3°± 1,5 (C = 1,009 %; Méthanol).

c) (amino-3 phényl)-1 éthanesulfonate d'hydroquinine, forme A :

A une solution de 10,4 g de (nitro-3 phényl)-1 éthanesulfonate de N-benzylquininium, forme A, dans 350 $cm^3$ d'éthanol, on ajoute 1,0 g de palladium sur noir à 5 %. La suspension est agitée pendant 2 heures à une température voisine de 25°C sous atmosphère d'hydrogène (100 kPa). Le catalyseur est séparé par filtration et le filtrat est concentré à sec sous pression réduite (2,7 kPa) à 40°C. Le produit obtenu est agité pendant 2 heures dans 100 $cm^3$ d'oxyde de diisopropyle. Le produit insoluble est séparé par filtration, lavé par 2 fois 20 $cm^3$ d'oxyde de diisopropyle et séché à l'air. On obtient ainsi 7,7 g d'(amino-3 phényl)-1 éthanesulfonate d'hydroquinine, forme A, sous forme d'une poudre utilisée telle quelle dans les synthèses ultérieures.

Spectre de R.M.N.: (200 MHz; $CDCl_3$; forme A) :
δ(ppm) :

| | |
|---|---|
| 0,75 | [t, J=7,5 Hz, 3H: -C$\underline{H}_3$ de (Q)] |
| de 1,65 à 2,10 | [mt, 4H: 1H du -C$\underline{H}_2$- en 5, 1H du -C$\underline{H}_2$- en 3, >C$\underline{H}$ en 4, >C$\underline{H}$ en 8 de (Q)] |
| de 2,40 à 2,75 | [mt, 2H: 1H du >N-(C$\underline{H}_2$-) en 6 et 1H du >N-(C$\underline{H}_2$-) en 7 de (Q)] |
| 3,15 | [mt, 1H: 1H du >N-(C$\underline{H}_2$-) en 7 de (Q)] |
| 3,32 | [mt, 1H: C$\underline{H}$ en 2 de (Q)] |
| 3,48 | [mt, 1H: 1H du >N-(C$\underline{H}_2$-) en 6 de (Q)] |
| 3,99 | [s, 3H: -OC$\underline{H}_3$ de (Q)] |
| 4,04 | [q, J=7 Hz, 1H: -C$\underline{H}$($CH_3$)-] |
| 5,69 | [s, 1H: -O-C(H)< en 9 de (Q)] |
| 6,48 | [mt, 1H: -$C_6H_4$ (-$\underline{H}$4)] |
| 6,93 | [mt, 2H: -$C_6H_4$ (-$\underline{H}$6) et (-$\underline{H}$5)] |
| 7,00 | [large, 1H: -$C_6H_4$ (-$\underline{H}$2)] |
| 7,17 | [d, J=2 Hz, 1H: (-$\underline{H}$5') de (Q)] |
| 7,31 | [dd, J=8,5 et 2,5 Hz, 1H: (-$\underline{H}$7') de (Q)] |
| 7,62 | [d, J=5 Hz, 1H: (-$\underline{H}$3') de (Q)] |
| 7,96 | [d, J=8,5 Hz, 1H: (-$\underline{H}$8') de (Q)] |
| 8,70 | [d, J=5 Hz, 1H: (-$\underline{H}$2') de (Q)] |

## EXEMPLE D'APPLICATION

A une solution de 5,3 g d'{[(imidazolyl-1) carboxamido]-2 N-(méthoxy-3 phényl) acétamido}-2 N-méthyl N-phényl-acétamide dans 360 $cm^3$ de toluène, on ajoute 7,7 g d'(amino-3 phényl)-1 éthanesulfonate d'hydroquinine, forme A, en solution dans 45 $cm^3$ de diméthylformamide. Le mélange réactionnel est agité à reflux pendant 5 heures, refroidi à environ 25°C et coulé dans 400 $cm^3$ d'eau. Le pH est ajusté à 8 avec une solution aqueuse de soude 4N. La solution aqueuse est lavée par 3 fois 300 $cm^3$ d'acétate d'éthyle puis on ajoute 30 g de dihydrogénophosphate de potassium et 4,6 g d'hydrogénosulfate de tétra-n-butyl ammonium. Le mélange est extrait par 3 fois 150 $cm^3$de chlorure de méthylène. Les phases organiques réunies sont séchées sur sulfate de magnésium et concentrées à sec sous pression réduite (2,7 kPa) à 40°C. Le produit brut obtenu est agité 30 minutes dans 100 $cm^3$ d'oxyde de diisopropyle. Le produit insoluble est séparé par filtration puis dissous dans 10 $cm^3$ d'acétone. On ajoute 2,6 g de nonafluorobutanesulfonate de potassium en solution dans 6 $cm^3$ d'acétone puis 5 $cm^3$ d'oxyde de diisopropyle. La gomme insoluble est séparée puis agitée pendant 2 heures dans 25 $cm^3$ d'un mélange d'acétone et d'oxyde de diisopropyle (60/40 en volumes). Le produit insoluble est séparé par filtration, puis dissous dans 10 $cm^3$ d'acétone. On ajoute 2,6 g de nonafluorobutanesulfonate de potassium en solution dans 6 $cm^3$ d'acétone puis 5 $cm^3$ d'oxyde de diisopropyle. La gomme insoluble est séparée puis agitée pendant 2 heures dans 25 $cm^3$ d'un mélange d'acétone et d'oxyde de diisopropyle (60/40 en volumes). Le produit insoluble est séparé par filtration, lavé par 2 fois 15 $cm^3$ d'un mélange d'acétone et d'oxyde de diisopropyle (60/40 en volumes) puis 4 fois 10 $cm^3$ d'oxyde de diisopropyle et séché à l'air. On obtient ainsi 4,1 g de (+)-{{[N-(méthoxy-3 phényl) N-(N-méthyl N-phényl-carbamoylméthyl) carbamoylméthyl]-3 uréido}-3 phényl}-1 éthanesulfonate de potassium fondant à environ 180°C. $[\alpha]_D^{20}$ = + 5,2°± 0,5 (C = 0,806 %; Méthanol).

Spectre de R.M.N.: (300 MHz; DMSO d6: forme (-))
d(ppm):

1,43 [d,J=7Hz,3H: -CH(C$\underline{H}_3$,)-]

3,18 [s large, 3H; -N(C$\underline{H}_3$)-]
3,60 [mt, 1H: -C$\underline{H}$($CH_3$)-]
3,65 [d large, J=5Hz, 2H: -CO(C$\underline{H}_2$)NH-]
3,79 (s, 3H: -OC$\underline{H}_3$)
4,09 [mf, 2H: -CO($CH_2$)N<]

6,28 [t large, J=5Hz, 1H: -N$\underline{H}$-]
6,86 [d,J=7.5 Hz, 1H: -$C_6H_4$(-H4) du N-(Methoxy-3 phényl)]

de 6,95 à 7,15 (mt, 4H: aromatiques)
7,17 (s large, 1H:-$C_6H_4$(-H2) du N-(méthoxy-3 phényl)]

de 7,30 à 7,50 (mt, 11H: aromatiques).
8,80 (s large, 1H: -CO-N$\underline{H}$-Ar).

L'{[(imidazolyl-1) carboxamido]-2 N-(méthoxy-3 phényl) acétamido}-2 N-méthyl N-phényl-acétamide peut être préparé de la manière suivante : à une solution de 3,0 g de N,N'-diimidazolecarbonyle dans 30 $cm^3$ de tétrahydrofuranne anhydre, on ajoute une solution de 3,1 g d'[amino-2 N-(méthoxy-3 phényl) acétamido]-2 N-méthyl N-phényl-acétamide dans 30 $cm^3$ de tétrahydrofuranne anhydre. La solution est agitée pendant 16 heures à une température voisine de 25°C puis concentrée à sec sous pression réduite (2,7 kPa) à 40°C. Le résidu est dissous dans 50 $cm^3$ d'acétate d'éthyle et la solution obtenue est lavée par 4 fois 30 $cm^3$ d'eau. La phase organique est séchée sur sulfate de magnésium puis concentrée à sec sous pression réduite (2,7 kPa) à 40°C. On obtient, après recristallisation dans l'acétate d'éthyle, 3,5 g d'{[(imidazolyl-1) carboxamido]-2 N-(méthoxy-3 phényl) acétamido}-2 N-méthyl N-phényl-acétamide fondant à 146°C

L'[amino-2 N-(méthoxy-3 phényl) acétamido]-2 N-méthyl N-phényl-acétamide peut être préparé de la manière suivante : à une solution de 5,5 g de [N-(méthoxy-3 phényl) phtalimido-2 acétamido]-2 N-méthyl N-phényl-acétamide dans 60 $cm^3$ de méthanol, on ajoute 1,3 g d'hydrate d'hydrazine. Le mélange réactionnel est agité à reflux pendant 30 minutes et, après refroidissement, on ajoute 100 $cm^3$ d'eau. Le mélange est concentré à environ 100 $cm^3$puis amené à pH 9 avec une solution aqueuse de soude 2N et extrait par 2 fois 50 $cm^3$ d'acétate d'éthyle. Les phases organiques réunies sont lavées par 2 fois 50 $cm^3$ d'eau, séchées sur sulfate de magnésium puis concentrées à sec sous pression réduite (2,7 kPa) à 40°C. On obtient ainsi 3,0 g d'[amino-2 N-(méthoxy-3 phényl) acétamido]-2 N-méthyl N-phényl-acétamide sous forme d'une huile utilisée telle quelle dans les synthèses ultérieures.

Le [N-(méthoxy-3 phényl) phtalimido-2 acétamido]-2 N-méthyl N-phényl-acétamide peut être préparé de la manière suivante : à une suspension de 80,6 g d'acide [N-(méthoxy-3 phényl) phtalimido-2 acétamido]-2 acétique dans 900 $cm^3$ de dichloro-1,2 éthane on ajoute 10 $cm^3$ de diméthylformamide puis en 1 heure, 30,2 g de dichlorure d'oxalyle. Le mélange est agité 2 heures à une température voisine de 25°C, puis on ajoute 58,6 g de N-méthyl aniline en 45 minutes. Le mélange réactionnel est agité 2 heures à une température voisine de 25°C, lavé par 2 fois 500 $cm^3$ d'eau puis 300 $cm^3$ d'une solution aqueuse saturée d'hydrogénocarbonate de sodium, séché sur sulfate de magnésium et concentré à sec sous pression réduite (2,7 kPa) à 40°C. Le résidu est agité une heure dans 300 $cm^3$ d'oxyde de diisopropyle, le produit insoluble est séparé par filtration, lavé par 3 fois 60 $cm^3$ d'oxyde de diisopropyle et séché à l'air. On obtient ainsi, 84 g de [N-(méthoxy-3 phényl) phtalimido-2 acétamido]-2 N-méthyl N-phényl-acétamide fondant à 137°C.

L'acide [N-(méthoxy-3 phényl) phtalimido-2 acétamido]-2 acétique peut être préparé de la manière suivante : à une solution de 42,0 g de [N-(méthoxy-3 phényl) phtalimido-2 acétamido]-2 acétate de tert-butyle dans 500 $cm^3$ de chlorure de méthylène, on ajoute 74,0 g d'acide trifluoroacétique. La solution obtenue est agitée à reflux pendant 5 heures, puis concentrée à sec sous pression réduite (2,7 kPa) à 40°C. Le résidu est agité pendant une heure dans 100 $cm^3$ d'oxyde de diisopropyle, le produit insoluble est séparé par filtration, lavé par 3 fois 40 $cm^3$ d'oxyde de diisopropyle et séché à l'air. On obtient ainsi, 36 g d'acide [N-(méthoxy-3 phényl) phtalimido-2 acétamido]-2 acétique fondant à 203°C.

Le [N-(méthoxy-3 phényl) phtalimido-2 acétamido]-2 acétate de tert-butyle peut être préparé de la manière suivante : à une solution de 96 g de N-(méthoxy-3 phényl) phtalimido-2 acétamide dans 1000 $cm^3$ de tétrahydrofuranne anhydre, on ajoute en 30 minutes 14,9 g d'une suspension huileuse (60 % en poids) d'hydrure de sodium. La suspension est agitée pendant 4 heures à une température voisine de 20°C puis on ajoute en 15 minutes 72,7 g de bromoacétate de tert-butyle. Le mélange réactionnel est agité pendant 16 heures à une température voisine de 25°C, hydrolysé lentement par 50 $cm^3$ d'eau, puis concentré à sec sous pression réduite. Le résidu obtenu est agité pendant une heure dans 400 $cm^3$ d'eau, le produit insoluble est séparé par filtration, lavé par 3 fois 100 $cm^3$ d'eau, 2 fois 100 $cm^3$ d'oxyde de diisopropyle et séché à l'air. On obtient ainsi, 82,0 g de [N-(méthoxy-3 phényl) phtalimido-2 acétamido]-2 acétate de tert-butyle fondant à 148°C.

Le N-(méthoxy-3 phényl) phtalimido-2 acétamide peut être préparé de la manière suivante : à une solution de 26,0 g de méthoxy-3 aniline dans 200 $cm^3$ de chlorure de méthylène, on ajoute 22,0 g de triéthylamine puis 48,0g de chlorure de phtalimido-2 acétyle en solution dans 300 $cm^3$ de chlorure de méthylène en maintenant la température à environ

20°C. Le mélange réactionnel est agité pendant 4 heures, à cette température, puis on ajoute 800 $cm^3$ d'eau. Le produit insoluble est séparé par filtration, lavé par 3 fois 100 $cm^3$ d'eau et séché à l'air. On obtient ainsi 65,0 g de N-(méthoxy-3 phényl) phtalimido-2 acétamide fondant à 171°C.

Le chlorure de phtalimido-2 acétyle peut être préparé selon la méthode décrite par W. GRASSMANN et E. SCHULTE-UEBBING, Chem. Ber.,83, 244-247, (1950).

## Revendications

**1.** L'(amino-3 phényl)-1 éthanesulfonate d'hydroquinine optiquement actif (Forme A) obtenable par le procédé comprenant les étapes suivantes :

a) action d'un sulfite de métal alcalin sur le (bromo-1 éthyl)-1 nitro-3 benzène-(RS) en milieu aqueux à une température comprise entre 50 et 100°C et passage au sel de potassium pour obtenir le (nitro-3 phényl)-1 éthanesulfonate de potassium-(RS),
b) conversion du (nitro-3 phényl)-1 éthanesulfonate de potassium-(RS) en (nitro-3 phényl)-1 éthanesulfonate de benzylquininium dont le point de fusion est d'environ 110°C et le pouvoir rotatoire $\alpha_D^{20}$ est de -151,3° ± 1,5 (C= 1,009 %; méthanol), au moyen d'un halogénure de N-benzyl quininium, en présence de dihydrogénophosphate de potassium, en milieu aqueux, à une température comprise entre 10 et 30°C et séparation du produit par recristallisation dans le propanol-2,
c) réduction du (nitro-3 phényl)-1 éthanesulfonate de benzylquininium forme A obtenu en b) au moyen moyen d'hydrogène sous pression, en présence d'un catalyseur, à une température comprise entre 10 °C et 30 °C.

**2.** Procédé de préparation de l'(amino-3 phényl)-1 éthanesulfonate d'hydroquinine optiquement actif (Forme A) caractérisé en ce qu'il comprend les étapes suivantes :

a) action d'un sulfite de métal alcalin sur le (bromo-1 éthyl)-1 nitro-3 benzène-(RS) en milieu aqueux à une température comprise entre 50 et 100°C et passage au sel de potassium pour obtenir le (nitro-3 phényl)-1 éthanesulfonate de potassium-(RS),
b) conversion du (nitro-3 phényl)-1 éthanesulfonate de potassium-(RS) en (nitro-3 phényl)-1 éthanesulfonate de benzylquininium dont le point de fusion est d'environ 110°C et le pouvoir rotatoire $\alpha_D^{20}$ est de -151,3° ± 1,5 (C= 1,009 %; méthanol), au moyen d'un halogénure de N-benzyl quininium, en présence de dihydrogénophosphate de potassium, en milieu aqueux, à une température comprise entre 10 et 30°C et séparation du produit par recristallisation dans le propanol-2,
c) réduction du (nitro-3 phényl)-1 éthanesulfonate de benzylquininium forme A obtenu en b) au moyen moyen d'hydrogène sous pression, en présence d'un catalyseur, à une température comprise entre 10 °C et 30 °C.

**3.** Procéde selon la revendication 2 pour lequel l'étape a) s'effectue à 80°C.

**4.** Procéde selon la revendication 2 pour lequel dans l'étape a) on récupère le produit en le transformant en sel de tétra-alkylammonium ou trialkylphénylalkylammonium puis en sel de potassium.

**5.** Procédé selon la revendication 2 pour lequel dans l'étape c) on opère sous une pression d'hydrogène de 100 kPa.

**6.** Utilisation du composé selon la revendication 1 pour la préparation de l'énantiomère actif des composés de formule :

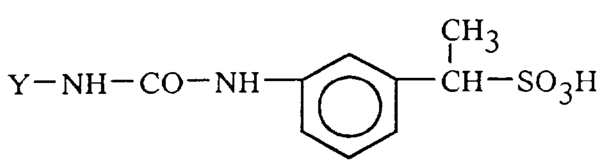

(I)

dans laquelle Y représente :

A) un reste $-CH_2-CO-NR_1R_2$ dans lequel $R_1$ représente un radical (a) phényle éventuellement substitué par un ou plusieurs substituants choisis parmi les atomes d'halogène et les radicaux alkyle, alcoxy, hydroxy polyfluoroalkyle, nitro, alkylthio, alcoxycarbonyle, carboxy, acylamino, méthylènedioxy, polyfluoroalcoxy, trifluorométhylthio, phénoxy, phényl, benzyle, phénylamino et $CONR_3R_4$ dans lequel $R_3$ et $R_4$, identiques ou différents, représentent un atome d'hydrogène, un radical alkyle, phényle (éventuellement substitué par un ou plusieurs

substituants choisis parmi les atomes d'halogène et les radicaux alkyle, alcoxy et alkylthio), indanyle, cycloalkylalkyle, cycloalkyle, phénylalkyle ou bien $R_3$ et $R_4$ forment ensemble avec l'atome d'azote auquel ils sont rattachés un hétérocycle mono ou polycyclique saturé ou insaturé contenant 4 à 9 atomes de carbone et un ou plusieurs hétéroatomes (O,N,S) et éventuellement substitué par un plusieurs radicaux alkyle, alcoxy, alcoxycarbonyle, dialkylcarbamoyle, phényle ou en combinaison avec un atome de carbone de l'hétérocycle par un cycle spiromonocyclique à 4 ou 5 chaînons et contenant éventuellement un ou plusieurs hétéroatomes (O,S,N) ; (b) pyridyle, (c) isoquinolyle, (d) quinolyle, (e) quinoxalinyle (ces hétérocycles étant éventuellement substitués par un ou plusieurs substituants choisis parmi les radicaux alkyle, phényle et les atomes d'halogène), (f) alkyle, (g) phénylalkyle, (h) naphtyle, (i) tétrahydro-5,6,7,8 naphtyle,(j) tétrahydro-1,2,3,4 naphtyle, (k) alcoxycarbonylalkyle ou (1) cycloalkyle,

$R_2$ représente un chaîne -CH($R_5$)-CO-$R_6$ dans laquelle $R_5$ représente un atome d'hydrogène ou un radical alkyle, alcoxycarbonyle ou phényle (éventuellement substitué par un ou plusieurs substituants choisis parmi les atomes d'halogène et les radicaux alkyle, alcoxy, alkylthio, nitro et amino) et $R_6$ représente un radical alcoxy, cycloalkyloxy (éventuellement substitué par au moins un radical alkyle), cycloalkylalkyloxy, phénylalkyloxy, polyfluoroalkyloxy, cinnamyloxy et -$NR_3R_4$,

B) un reste de formule :

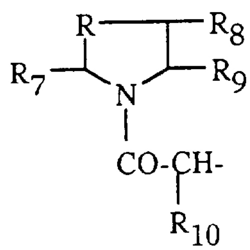

dans laquelle :

- soit R représente un radical méthylène, éthylène, SO, $SO_2$, CHOH ou un atome de soufre, $R_7$ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-$NR_{11}R_{12}$, -NH-CO-$CH_3$, trifluorométhyle ou trifluorométhoxy et $R_8$ représente un atome d'hydrogène,
- soit R représente un radical méthylène, $R_7$ représente un atome d'hydrogène et $R_8$ représente un radical phényle,
- soit R représente un radical $CHR_{13}$, $R_7$ et $R_8$ représentent chacun un atome d'hydrogène,
- $R_9$ représente un radical alcoxycarbonyle, cycloalkyloxycarbonyle, cyclo-alkylalkyloxycarbonyle, -$CONR_{14}R_{15}$ ou phényle éventuellement substitué par un ou plusieurs substituants choisis parmi les radicaux alkyle, alcoxy ou hydroxy,
- $R_{10}$ représente un atome d'hydrogène ou un radical alkyle,
- $R_{13}$ représente un radical phényle,
- $R_{11}$ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- $R_{12}$ représente un radical alkyle, phénylalkyle ou phényle éventuellement substitué par un ou plusieurs substituants choisis parmi les atomes d'halogène et les radicaux alkyle, alcoxy et alkylthio,

ou bien $R_{11}$ et $R_{12}$ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,

- $R_{14}$ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,

- $R_{15}$ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,

ou bien $R_{14}$ et $R_{15}$ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,

C) un reste de formule :

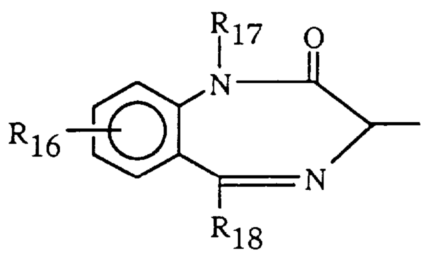

dans laquelle :

- $R_{16}$ représente un atome d'hydrogène ou d'halogène ou un radical alkyle, alkylthio, nitro, hydroxy ou cyano,
- $R_{17}$ représente un radical alkyle ou un reste -CH($R_5$)-CO-$R_6$,
- $R_{18}$ représente un radical pyridyle ou phényle éventuellement substitué par un ou plusieurs substituants choisis parmi les atomes d'halogène et les radicaux alkyle, alcoxy, hydroxy, carboxy et nitro.

## Patentansprüche

**1.** Optisch aktives Hydrochinin-1-(3-aminophenyl)ethansulfonat (Form A), erhältlich durch das Verfahren, das die folgenden Schritte umfaßt:

a) Einwirkung eines Alkalimetallsulfits auf 1-(1-Bromethyl) -3-nitrobenzol-(RS) in wäßrigem Medium bei einer Temperatur zwischen 50 und 100°C eingeschlossen und Überführung in das Kaliumsalz, um Kalium-1-(3-nitrophenyl)ethansulfonat-(RS) zu erhalten,
b) Überführen von Kalium-1-(3-nitrophenyl) -ethansulfonat-(RS) in Benzylchininium-1-(3-nitrophenyl)ethansulfonat, dessen Schmelzpunkt bei ungefähr 110°C liegt und dessen Drehvermögen $\alpha_D^{20}$ -151,3° ± 1,5 (C = 1,009%; Methanol) beträgt, mittels eines N-Benzylchininiumhalogenids in Gegenwart von Kaliumdihydrogenphosphat in wäßrigem Medium bei einer Temperatur zwischen 10 und 30°C eingeschlossen und Abtrennen des Produkts durch Umkristallisation aus 2-Propanol,
c) Reduktion des Benzylchininium-1-(3-nitrophenyl)ethansulfonats, Form A, erhalten in b), mittels Wasserstoff unter Druck in Gegenwart eines Katalysators bei einer Temperatur zwischen 10°C und 30°C eingeschlossen.

**2.** Verfahren zur Herstellung von optisch aktivem Hydrochinin-1-(3-aminophenyl)ethansulfonat (Form A), dadurch gekennzeichnet, daß es die folgenden Schritte umfaßt:

a) Einwirkung eines Alkalimetallsulfits auf 1-(1-Bromethyl) -3-nitrobenzol-(RS) in wäßrigem Medium bei einer Temperatur zwischen 50 und 100°C eingeschlossen und Überführung in das Kaliumsalz, um Kalium-1-(3-nitrophenyl)ethansulfonat-(RS) zu erhalten,
b) Überführen von Kalium-1-(3-nitrophenyl)ethansulfonat-(RS) in Benzylchininium-1-(3-nitrophenyl)ethansulfonat, dessen Schmelzpunkt bei ungefähr 110°C liegt und dessen Drehvermögen $\alpha_D^{20}$ -151,30° ± 1,5 (C =

1,009%; Methanol) beträgt, mittels eines N-Benzylchininiumhalogenids in Gegenwart von Kaliumdihydrogenphosphat in wäßrigem Medium bei einer Temperatur zwischen 10 und 30°C eingeschlossen und Abtrennen des Produkts durch Umkristallisation aus 2-Propanol,

c) Reduktion des Benzylchininium-1-(3-nitrophenyl)ethansulfonats, Form A, erhalten in b), mittels Wasserstoff unter Druck in Gegenwart eines Katalysators bei einer Temperatur zwischen 10°C und 30°C eingeschlossen.

**3.** Verfahren nach Anspruch 2, bei dem der Schritt a) bei 80°C vorgenommen wird.

**4.** Verfahren nach Anspruch 2, bei dem man in dem Schritt a) das Produkt gewinnt, indem man es in ein Tetraalkylammonium- oder Trialkylphenylalkylammoniumsalz, dann in das Kaliumsalz überführt.

**5.** Verfahren nach Anspruch 2, bei dem man in dem Schritt c) unter einem Wasserstoffdruck von 100 kPa arbeitet.

**6.** Verwendung der Verbindung nach Anspruch 1 bei der Herstellung des aktiven Enantiomers der Verbindungen der Formel:

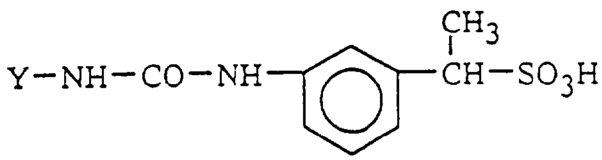

(I)

in der Y darstellt:

A) einen Rest $-CH_2-CO-NR_1R_2$, in dem $R_1$ einen (a) Phenylrest, der gegebenenfalls mit einem oder mehreren Substituenten substituiert ist, die ausgewählt sind aus den Halogenatomen und Alkyl-, Alkoxy-, Hydroxy-, Polyfluoroalkyl-, Nitro-, Alkylthio-, Alkoxycarbonyl-, Carboxy-, Acylamino-, Methylendioxy-, Polyfluoralkoxy-, Trifluormethylthio-, Phenoxy-, Phenyl-, Benzyl-, Phenylaminoresten und $CONR_3R_4$, worin $R_3$ und $R_4$, identisch oder verschieden, ein Wasserstoffatom, einen Alkyl-, Phenylrest (gegebenenfalls mit einem oder mehreren Substituenten substituiert, die ausgewählt sind aus den Halogenatomen und Alkyl-, Alkoxy- und Alkylthioresten), Indanyl-, Cycloalkylalkyl-, Cycloalkyl-, Phenylalkylrest darstellen oder auch $R_3$ und $R_4$ zusammen mit dem Stickstoffatom, an das sie gebunden sind, einen mono- oder polycyclischen gesättigten oder ungesättigten Heterocyclus mit 4 bis 9 Kohlenstoffatomen und einem oder mehreren Heteroatomen (O,N,S) bilden, der gegebenenfalls mit einem oder mehreren Alkyl-, Alkoxy-, Alkoxycarbonyl-, Dialkylcarbamoyl-, Phenylresten oder in Kombination mit einem Kohlenstoffatom des Heterocyclus mit einem monocyclischen Spirocyclus mit 4 oder 5 Ringgliedern, der gegebenenfalls ein oder mehrere Heteroatome (O,S,N) enthält, substituiert ist; (b) Pyridyl-, (c) Isochinolyl-, (d) Chinolyl-, (e) Chinoxalinylrest (wobei diese Heterocyclen gegebenenfalls mit einem oder mehreren Substituenten substituiert sind, die aus Alkyl-, Phenylresten und den Halogenatomen ausgewählt sind), (f) Alkyl-, (g) Phenylalkyl-, (h) Naphthyl-, (i) Tetrahydro-5,6,7,8-naphthyl-, (j ) Tetrahydro-1,2,3,4-naphthyl-, (k) Alkoxycarbonylalkyl- oder (1) Cycloalkylrest darstellt,

$R_2$ eine Kette $-CH(R_5)-CO-R_6$ darstellt, in der $R_5$ ein Wasserstoffatom oder einen Alkyl-, Alkoxycarbonyl-oder Phenylrest (gegebenenfalls mit einem oder mehreren Substituenten substituiert, die aus den Halogenatomen und Alkyl-, Alkoxy-, Alkylthio-, Nitro- und Aminoresten ausgewählt sind) darstellt und $R_6$ einen Alkoxy-, Cycloalkyloxyrest (gegebenenfalls mit mindestens einem Alkylrest substituiert), Cycloalkylalkyloxy-, Phenylalkyloxy-, Polyfluoralkyloxy-, Cinnamyloxyrest und $-NR_3R_4$ darstellt,

B) einen Rest der Formel:

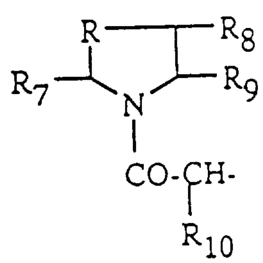

in der:

- entweder R einen Methylen-, Ethylenrest, SO, $SO_2$, CHOH oder ein Schwefelatom darstellt, $R_7$ einen Pyridylrest, der gegebenenfalls mit einem oder mehreren Alkylresten substituiert ist, Furylrest, der gegebenenfalls mit einem oder mehreren Alkylresten substituiert ist, Thienylrest, der gegebenenfalls mit einem oder mehreren Alkylresten substituiert ist, Chinolylrest, der gegebenenfalls mit einem oder mehreren Alkylresten substituiert ist, Naphthylrest, der gegebenenfalls mit einem oder mehreren Alkylresten substituiert ist, Indolylrest, der gegebenenfalls mit einem oder mehreren Alkylresten substituiert ist, oder Phenylrest darstellt, der gegebenenfalls mit einem oder mehreren Substituenten substituiert ist, die aus den Halogenatomen und Alkyl-, Alkoxy-, Hydroxy-, Nitro-, Amino-, Monoalkyl-amino-, Dialkylamino-, Alkoxycarbonylresten, $-CO-NR_{11}R_{12}$, $-NH-CO-CH_3$, Trifluormethyl- oder Trifluormethoxyresten ausgewählt sind, und $R_8$ ein Wasserstoffatom darstellt,

- oder R einen Methylenrest darstellt, $R_7$ ein Wasserstoffatom darstellt und $R_8$ einen Phenylrest darstellt,

- oder R einen Rest $CHR_{13}$ darstellt, $R_7$ und $R_8$ jeweils ein Wasserstoffatom darstellen,

- $R_9$ einen Alkoxycarbonyl-, Cycloalkyloxy-carbonyl-, Cycloalkylalkyloxycarbonylrest, $-CONR_{14}R_{15}$ oder einen Phenylrest darstellt, der gegebenenfalls mit einem oder mehreren Substituenten substituiert ist, die aus Alkyl-, Alkoxy- oder Hydroxyresten ausgewählt sind,

- $R_{10}$ ein Wasserstoffatom oder einen Alkylrest darstellt,

- $R_{13}$ einen Phenylrest darstellt,

- $R_{11}$ ein Wasserstoffatom oder einen Alkyl-, Phenylalkyl- oder Phenylrest darstellt, der gegebenenfalls mit einem oder mehreren Substituenten substituiert ist, die aus den Halogenatomen und Alkyl-, Alkoxy- und Alkylthioresten ausgewählt sind,

- $R_{12}$ einen Alkyl-, Phenylalkyl- oder Phenylrest darstellt, der gegebenenfalls mit einem oder mehreren Substituenten substituiert ist, die aus den Halogenatomen und Alkyl-, Alkoxy- und Alkylthioresten ausgewählt sind,

- oder auch $R_{11}$ und $R_{12}$ zusammen mit dem Stickstoffatom, an das sie gebunden sind, einen mono- oder polycyclischen gesättigten oder ungesättigten Heterocyclus mit 4 bis 9 Kohlenstoffatomen und einem oder mehreren Heteroatomen (O,N) bilden, der gegebenenfalls mit einem oder mehreren Alkylresten substituiert ist,

- $R_{14}$ ein Wasserstoffatom oder einen Alkyl-, Cycloalkylalkyl-, Cycloalkyl-, Phenylalkyl-oder Phenylrest darstellt, der gegebenenfalls mit einem oder mehreren Substituenten substituiert ist, die aus den Halogenatomen und Alkyl-, Alkoxy- und Alkylthioresten ausgewählt sind,

- $R_{15}$ einen Alkyl-, Cycloalkylalkyl-, Cycloalkyl-, Phenylalkyl- oder Phenylrest darstellt, der gegebenenfalls mit einem oder mehreren Substituenten substituiert ist, die aus den Halogenatomen und Alkyl-, Alkoxy- und Alkylthioresten ausgewählt sind,

- oder auch $R_{14}$ und $R_{15}$ zusammen mit dem Stickstoffatom, an das sie gebunden sind, einen mono- oder polycyclischen gesättigten oder ungesättigten Heterocyclus mit 4 bis 9 Kohlenstoffatomen und einem oder mehreren Heteroatomen (O,N,S) bilden, der gegebenenfalls mit einem oder mehreren Alkylresten substituiert ist,

C) einen Rest der Formel:

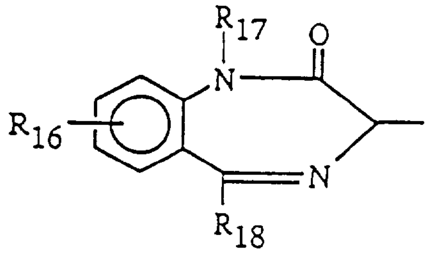

in der:

- $R_{16}$ ein Wasserstoff- oder Halogenatom oder einen Alkyl-, Alkylthio-, Nitro-, Hydroxy- oder Cyanorest darstellt,
- $R_{17}$ einen Alkylrest oder einen Rest $-CH(R_5)-CO-R_6$ darstellt,
- $R_{18}$ einen Pyridyl- oder Phenylrest darstellt, der gegebenenfalls mit einem oder mehreren Substituenten substituiert ist, die aus den Halogenatomen und Alkyl-, Alkoxy-, Hydroxy-, Carboxy- und Nitroresten ausgewählt sind.

**Claims**

**1.** Optically active hydroquinine 1- (3-aminophenyl) - ethanesulphonate (Form A) obtainable by the process comprising the following stages:

a) action of an alkali metal sulphite on (RS)-1- (1-bromoethyl) -3-nitrobenzene in aqueous medium at a temperature of between 50 and 100°C and passage to the potassium salt in order to obtain potassium (RS) -1 - (3 nitrophenyl) ethanesulphonate,
b) conversion of the potassium (RS)-1-(3-nitrophenyl)-ethanesulphonate to benzylquininium 1- (3-nitro-phenyl) ethanesulphonate whose melting point is about 110°C and whose optical rotation $[\alpha]_D^{20}$ is -151.3° ± 1.5 (C = 1.009%; methanol), by means of an N-benzylquininium halide, in the presence of potassium dihydrogen phosphate, in aqueous medium, at a temperature of between 10 and 30°C and separation of the product by recrystallization from 2-propanol,
c) reduction of the benzylquininium 1- (3-nitrophenyl)-ethanesulpbonate Form A obtained in b) by means of hydrogen under pressure, in the presence of a catalyst, at a temperature of between 10°C and 30°C.

**2.** Process for the preparation of optically active hydroquinine 1- (3-aminophenyl) ethanesulphonate (Form A), characterized in that it comprises the following stages:

a) action of an alkali metal sulphite on (RS)-1- (1-bromoethyl) -3-nitrobenzene in aqueous medium at a temperature of between 50 and 100°C and passage to the potassium salt in order to obtain potassium (RS)-1-(3-nitrophenyl) ethanesulphonate,
b) conversion of the potassium (RS) -1- (3-nitrophenyl)-ethanesulphonate to benzylquininium 1-(3-nitrophenyl) ethanesulphonate whose melting point is about 110°C and whose optical rotation $[\alpha]_D^{20}$ is -151.3 ° ± 1.5 (C = 1.009%; methanol), by means of an N-benzylquininium halide, in the presence of potassium dihydrogen phosphate, in aqueous medium, at a temperature of between 10 and 30°C and separation of the product by recrystallization from 2-propanol,
c) reduction of the benzylquininium 1-(3-nitrophenyl)-ethanesulphonate Form A obtained in b) by means of hydrogen under pressure, in the presence of a catalyst, at a temperature of between 10°C and 30°C.

**3.** Process according to claim 2, in the case of which stage a) is performed at 80°C.

**4.** Process according to claim 2, in the case of which in stage a) the product is recovered by being converted into a

tetraalkylammonium or trialkylphenyl-alkylammonium salt and then into a potassium salt.

**5.** Process according to claim 2, in the case of which in stage c) the operation is carried out at a hydrogen pressure of 100 kPa.

**6.** Use of the compound according to claim 1 for the preparation of the active enantiomer of compounds of formula:

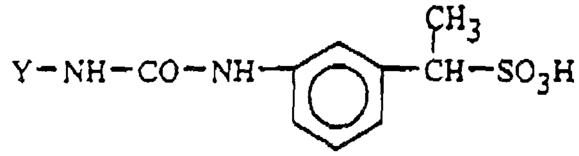

(I)

in which Y denotes:

A) a $-CH_2-CO-NR_1R_2$ residue in which $R_1$ denotes a radical which is (a) phenyl optionally substituted by one or more substituents chosen from halogen atoms and alkyl, alkoxy, hydroxyl, polyfluoroalkyl, nitro, alkylthio, alkoxycarbonyl, carboxyl, acylamino, methylenedioxy, polyfluoroalkoxy, trifluoromethylthio, phenoxy, phenyl, benzyl, phenyl-amino and $CONR_3R_4$, radicals in which $R_3$ and $R_4$, which are identical or different, denote a hydrogen atom, an alkyl, phenyl (optionally substituted by one or more substituents chosen from halogen atoms and alkyl, alkoxy and alkylthio radicals), indanyl, cycloalkylalkyl, cycloalkyl or phenylalkyl radical or else $R_3$ and $R_4$ together with the nitrogen atom to which they are attached form a saturated or unsaturated mono- or polycyclic heterocyclic ring containing 4 to 9 carbon atoms and one or more heteroatoms (O, N, S) and optionally substituted by one or more alkyl, alkoxy, alkoxycarbonyl, dialkylcarbamoyl or phenyl radicals or in combination with a carbon atom of the heterocyclic ring by a 4- or 5-membered spiromonocyclic ring optionally oontaining one or more heteroatoms (O, S, N); (b) pyridyl, (c) isoquinolyl, (d) quinolyl, (e) quinoxalinyl (these heterocyclic rings being optionally substituted by one or more substituents chosen from alkyl and phenyl radicals and halogen atoms), (f) alkyl, (g) phenylalkyl, (h) naphthyl, (i) 5,6,7,8-tetrahydronaphthyl, (j) 1,2,3,4-tetrahydronaphthyl, (k) alkoxycarbonylalkyl or (l) cycloalkyl,

$R_2$ denotes a $-CH(R_5)-CO-R_6$ chain in which $R_5$ denotes a hydrogen atom or an alkyl, alkoxycarbonyl or phenyl radical (optionally substituted by one or more substituents chosen from halogen atoms and alkyl, alkoxy, alkylthio, nitro and amino radicals) and $R_6$ denotes an alkoxy, cycloalkyloxy (optionally substituted by at least one alkyl radical), cycloalkylalkyloxy, phenylalkyloxy, polyfluoroalkyloxy, cinnamyloxy and $-NR_3R_4$ radical,

B) a residue of formula;

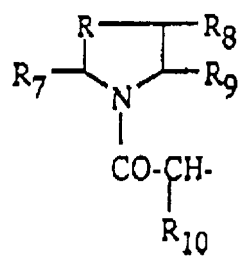

in which:

- either R denotes a methylene, ethylene, SO, $SO_2$ or CHOH radical or a sulphur atom, $R_7$ denotes a pyridyl radical optionally substituted by one or more alkyl radicals, furyl optionally substituted by one or more alkyl radicals, thienyl optionally substituted by one or more alkyl radicals, quinolyl optionally substituted by one or more alkyl radicals, naphthyl optionally substituted by one or more alkyl radicals, indolyl optionally substituted by one or more alkyl radicals or phenyl optionally substituted by one or more substituents chosen from halogen atoms and alkyl, alkoxy, hydroxyl, nitro, amino, monoalkylamino, dialkylamino, alkoxycarbonyl, $-CO-NR_{11}R_{12}$, $-NH-CO-CH_3$, trifluoromethyl or trifluoromethoxy radicals and $R_8$ denotes a hydrogen atom,
- or R denotes a methylene radical, $R_7$ denotes a hydrogen atom and $R_8$ denotes a phenyl radical,
- or R denotes a radical $CHR_{13}$, each of $R_7$ and $R_8$ denotes a hydrogen atom,
- $R_9$ denotes an alkoxycarbonyl, cycloalkyloxycarbonyl, cycloalkylalkyloxycarbonyl, $-CONR_{14}R_{15}$ or phenyl radical optionally substituted by one or more substituents chosen from alkyl, alkoxy or hydroxyl radicals,
- $R_{10}$ denotes a hydrogen atom or an alkyl radical,
- $R_{13}$ denotes a phenyl radical,

- $R_{11}$ denotes a hydrogen atom or an alkyl, phenylalkyl or phenyl radical optionally substituted by one or more substituents chosen from halogen atoms and alkyl, alkoxy and alkylthio radicals,
- $R_{12}$ denotes an alkyl, phenylalkyl or phenyl radical optionally substituted by one or more substituents chosen from halogen atoms and alkyl, alkoxy and alkylthio radicals,

or else $R_{11}$ and $R_{12}$ with the nitrogen atom to which they are attached form a saturated or unsaturated mono- or polycyclic heterocyclic ring containing 4 to 9 carbon atoms and one or more heteroatoms (O,N) and optionally substituted by one or more alkyl radicals,

- $R_{14}$ denotes a hydrogen atom or an alkyl, cycloalkyl-alkyl, cycloalkyl, phenylalkyl or phenyl radical optionally substituted by one or more substituents chosen from halogen atoms and alkyl, alkoxy and alkylthio radicals,
- $R_{15}$ denotes an alkyl, cycloalkylalkyl, cycloalkyl, phenylalkyl or phenyl radical optionally substituted by one or more substituents chosen from halogen atoms and alkyl, alkoxy and alkylthio radicals,

or else $R_{14}$ and $R_{15}$ together with the nitrogen atom to which they are attached form a saturated or unsaturated mono- or polycyclic heterocyclic ring containing 4 to 9 carbon atoms and one or more heteroatoms (O,N,S) and optionally substituted by one or more alkyl radicals,
C) a residue of formula:

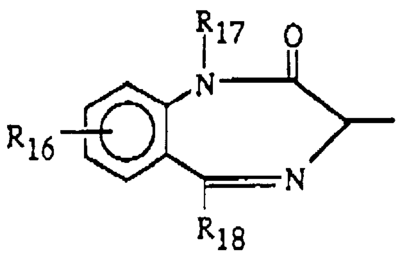

in which:

- $R_{16}$ denotes a hydrogen or halogen atom or an alkyl, alkylthio, nitro, hydroxyl or cyano radical,
- $R_{17}$ denotes an alkyl radical or a -CH($R_5$)-CO-$R_6$ residue,
- $R_{18}$ denotes a pyridyl or phenyl radical optionally substituted by one or more substituents chosen from halogen atoms and alkyl, alkoxy, hydroxyl, carboxyl and nitro radicals.